# EUROPEAN PATENT APPLICATION

(11) **EP 2 272 456 A1**
(43) Date of publication of application: **12.01.2011**
(21) Application number: 10161502.9
(22) Date of filing: 29.04.2010
(51) Int. Cl.: A61B 19/00, A61N 5/10

(54) **Localization using non-metallic implantable fiducial markers**

(30) Priority: 09.07.2009 US 500000
(71) Applicant: SIEMENS AKTIENGESELLSCHAFT, 80333 München (DE)
(72) Inventor: Bani-Hashemi, Ali-Reza, Walnut Creek, 94595 (US); Maltz, Jonathan S., Oakland, 94611 (US)

(57) **Abstract**

A system may include a biocompatible housing and an electrical circuit disposed within the housing, the electrical circuit to emit a radiofrequency signal, and wherein the electrical circuit does not comprise a metal. Also included may be a signal generator to generate a wireless signal to trigger the electrical circuit, a receiver to receive a wireless response signal generated by the triggered electrical circuit, a processor to determine a location of the biocompatible housing based on the wireless response signal, and a ion beam source to deliver a ion beam to a patient volume including the biocompatible housing.

## Description

### BACKGROUND

The embodiments described below relate generally to systems for delivering ion beam therapy. More specifically, some embodiments are directed to treatment verification systems used in conjunction with such delivery.

### Description

Conventional radiation therapy systems direct a beam of protons, x-ray photons, neutrons or positive ions to a target within a patient volume. The radiation dose delivered by the beam kills tissue cells within the target by damaging the DNA thereof. The radiation dose delivered by an x-ray beam, for example, is greatest at shallow tissue depths and decays exponentially as the depth increases. Accordingly, x-ray beams are suitable to kill cells (e.g., tumor cells) located at shallow tissue depths.

Various systems are used to ensure that a target is located in the path of an x-ray beam and that sensitive structures are avoided in accordance with a treatment plan. In one conventional example described in U.S. Patent Nos. 6,812,842 and 7,289,839, transponders are implanted within a patient. The transponders emit signals which are captured and analyzed to determine their locations with respect to an x-ray source. If the transponders are implanted within an organ or tumor, such systems are able to determine the location and/or orientation of the organ/tumor with respect to the x-ray source and to thereby determine whether or not the location/orientation corresponds to a treatment plan.

For therapeutic ion beams, the delivered dose increases with increasing tissue depth, up to a depth known as the Bragg peak. At depths greater than the Bragg peak, the dose drops to zero (for protons) or almost zero (for heavier ions). The location of the Bragg peak depends on factors including, but not limited to, the type of ion, the energy of the ion beam and the type of intervening tissue.

The present inventors have recognized that implantable transponders are incompatible with ion beam delivery systems. Particularly, the transponders contain metallic or other high-atomic-number materials (e.g., ferrites and copper) which affect the location of a Bragg peak and, as a result, the realized dose distribution.

### SUMMARY

In order to address the foregoing, some embodiments provide a biocompatible housing, an electrical circuit disposed within the housing, the electrical circuit to emit a radiofrequency signal, and wherein the electrical circuit does not comprise a metal. In some aspects, also included are a signal generator to generate a wireless signal to trigger the electrical circuit, a receiver to receive a response signal generated by the triggered electrical circuit, a processor to determine a location of the biocompatible housing based on the response signal, and a ion beam source to deliver a ion beam associated with the treatment plan.

According to some aspects, the processor is to determine a difference between the wireless response signal and a previous wireless response signal received from the electrical circuit, and to determine that the difference exceeds a threshold. In response to the determination, an imaging system is to generate an image of a patient volume in which the biocompatible housing is located, and a positioning system is to change a position of the patient volume with respect to a ion beam source based on the image. Alternatively, the positioning system is to change one or more characteristics of a ion beam to be delivered by a ion beam source based on the image.

The appended claims are not limited to the disclosed embodiments, however, as those in the art can readily adapt the descriptions herein to create other embodiments and applications.

### BRIEF DESCRIPTION OF THE DRAWINGS

Embodiments will become readily apparent from consideration of the following specification as illustrated in the accompanying drawings, in which like reference numerals designate like parts, and wherein:
FIG. 1A is a schematic diagram of an implantable marker according to some embodiments;
FIG. 1B is a schematic diagram of an implantable marker according to some embodiments;
FIG. 2 illustrates operation of a system according to some embodiments;
FIG. 3 is a flow diagram of process steps pursuant to some embodiments;
FIG. 4 is a perspective view of a treatment room according to some embodiments; and
FIG. 5 is a flow diagram of process steps pursuant to some embodiments.

### DETAILED DESCRIPTION

The following description is provided to enable a person in the art to make and use some embodiments and sets forth the best mode contemplated by the inventors for carrying out some embodiments. Various modifications, however, will remain readily apparent to those in the art.

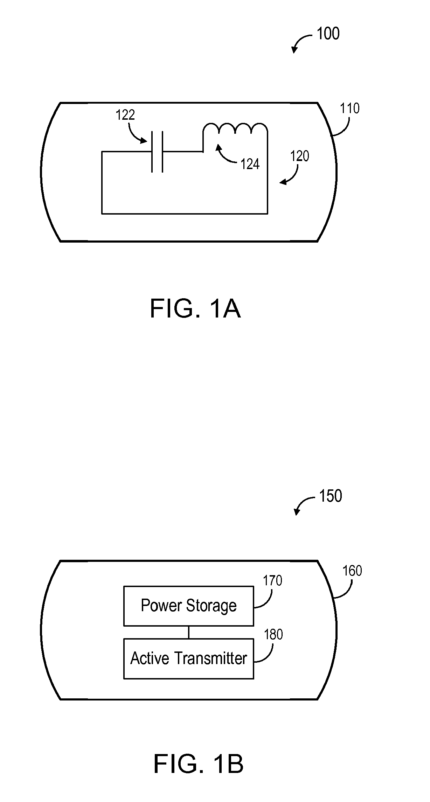

FIG. 1A is a schematic diagram of implantable marker 100 according to some embodiments. Implantable marker 100 comprises biocompatible housing 110 and electrical circuit 120 disposed in housing 110. Electrical circuit 120 is capable of emitting a radiofrequency signal, and does not comprise a metal. Other non-metallic elements may be disposed within housing 110 in addition to electrical circuit 110 according to some embodiments.

Biocompatible housing 110 may comprise one or more of a resin, plastic, ceramic, glass, and other biocompatible material that is or becomes known. Electrical circuit 120 may comprise carbon, aluminum, and/or any other suitable non-metallic material that is or becomes known. By virtue of the foregoing features, marker 100 may facilitate the positioning of a patient volume before or during delivery of a ion beam while only negligibly affecting a Bragg peak (and resulting dose distribution) associated with the ion beam.

Electrical circuit 120 of FIG. 1A comprises capacitor 122 electrically coupled to inductor 124. As such, electrical circuit 120 comprises an LC resonator exhibiting a resonant frequency determined by the values of capacitor 122 and inductor 124. Capacitor 122 and inductor 124 may each be implemented by one or more elements and in any suitable manner to provide a resonance response as described herein. Electrical circuit 120 may comprise non-metallic elements in addition to capacitor 122 and inductor 124.

Inductor 124 comprises a carbon nanocoil in some embodiments. The low resistance of a carbon nanocoil may result in a high quality factor (i.e., Q) of electrical circuit 120. As will be evident from the description of operation provided below, high Q may be desirable because Q is directly proportional to the amplitude of the resonant peak and inversely proportional to the width of the resonant peak of electrical circuit 120. In another example, inductor 124 may be fabricated on a semiconductor substrate as described in U.S. Patent No, 7,531,887. Capacitor 122 may comprise a conventional non-metallic surface mount capacitor, a capacitor based on carbon nanotubes or graphene sheets, and any other suitable type of capacitor. For example, large capacitance "supercapacitors" have been implemented based on carbon nanotube technology. Large capacitances allow for smaller inductances of inductor 124 to achieve a given resonant frequency.

According to one particular example, inductor 124 is a carbon nanocoil inductor comprising 600 turns, a 300nm diameter, a 100nm pitch and a 50 micron length. In free space, such a structure exhibits an inductance of 0.533nH. Capacitor 122, according to the present example, exhibits a capacitance of 500nF. The resulting resonant frequency is 9.75MHz. Notably, RF radiation in this range passes readily through body tissue. In yet another possibility which provides the above-described advantages of high Q and high resonant frequency, electrical circuit 120 may comprises a MEMS (i.e., a mechanical) resonator operating at high frequencies (e.g., GHz) and exhibiting high Q.

FIG. 1B is a block diagram of marker 150 according to some embodiments. Marker 150 comprises biocompatible housing 160, in which is disposed power storage 170 and active transmitter 180. Again, the materials of marker 150 do not include a metal, or contain negligible amounts of metal. For example, active transmitter 180 may comprise circuits fabricated on a low-density and low-atomic-number silicon substrate. Power storage 170 may comprise a charged supercapacitor which can be recharged inductively from an external source while implanted within a patient volume.

Active transmitter 180 may comprise a continuously-running oscillator, or an oscillator which switches on in response to an external RF signal for a predetermined period or until a switch-off pulse is received. Active transmitter 180 may emit pulse trains (e.g., pseudorandom sequences) that may be used to determine the location of marker 150.

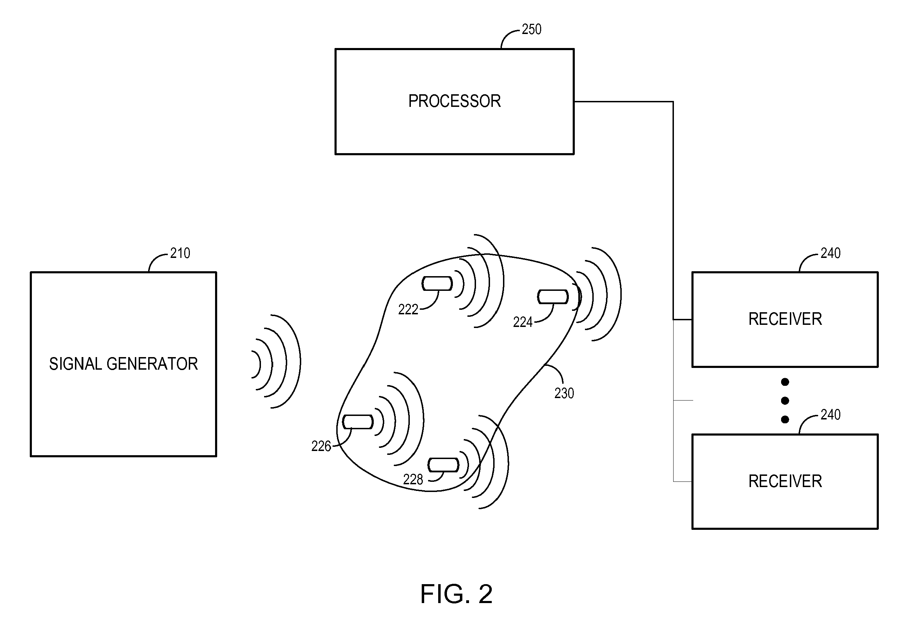

FIG. 2 illustrates operation of system 200 according to some embodiments. System 200 includes signal generator 210 to generate a wireless signal to trigger electrical circuits within markers 222 through 226 disposed in patient volume 230. Initially, it will be assumed that markers 222 through 226 comprise implementations of above-described marker 100. It will also be assumed that each of markers 222 through 226 includes an electrical circuit associated with a unique resonant frequency. If the Q of the electrical circuits is high, the unique resonant frequencies need not be significantly spaced across the applicable frequency spectrum.

The wireless signal generated by signal generator 210 includes a significant spectral component corresponding to the resonant frequency of each of markers 222 through 228. Accordingly, each of markers 222 through 228 emits a wireless response signal as illustrated in FIG. 2.

Each of receivers 240 measures a time delay between the end (or beginning) of the trigger signal and the end (or beginning) of the wireless response signal of each of markers 222 through 228. Because the locations of signal generator 210 and receivers 240 are known, processor 250 may determine the location of a particular marker based on the time delay measured by each receiver 240 for the particular marker.

In a case that markers 222 through 228 comprise implementations of marker 150, signal generator 210 may generate a trigger signal which causes active transmitters within markers 222 through 228 to emit unique and predetermined pulse trains. Receivers 240 receive the pulse trains and processor 250 may determine a location of one of markers 222 through 228 based on the difference in the reception time of a particular subsequence of its unique pulse train at two or more receivers (e.g., by procedures such as cross-correlation of the received and reference pulse trains).

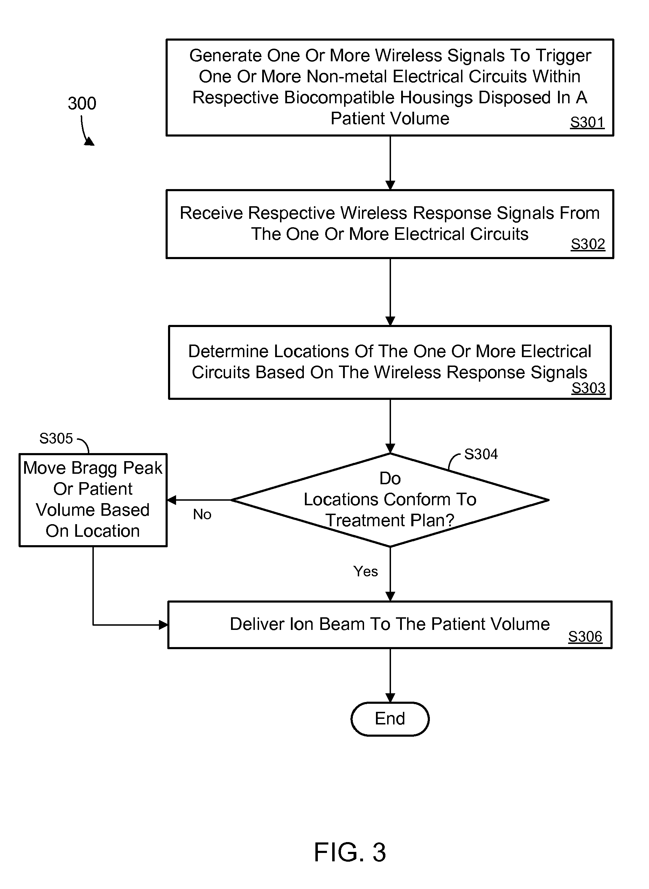

FIG. 3 is a flow diagram of a process according to some embodiments. Process 300 and the other processes described herein may be performed using any suitable combination of hardware, software or manual means. Software embodying these processes may be stored by any one or more tangible media, including but not limited to a fixed disk, a floppy disk, a CD-ROM, a DVD-ROM, a Zip™ disk, and a magnetic tape.

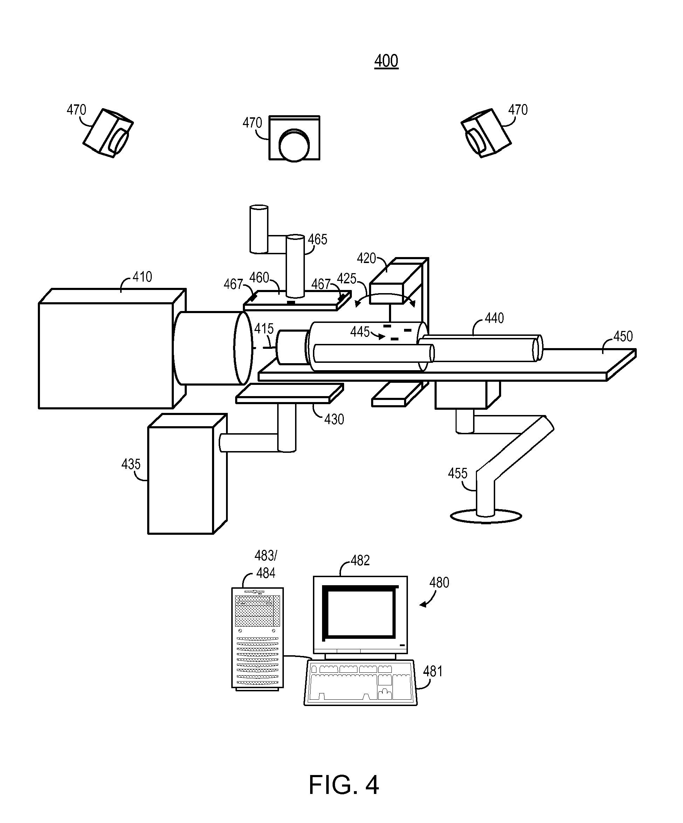

Process 300 may be performed after a patient has been placed on a treatment table in anticipation of ion therapy. FIG. 4 is a perspective view of treatment room 400 in which process 300 may occur according to some embodiments. Although process 300 will be described in conjunction with the elements of FIG. 4, embodiments are not limited to the illustrated elements or arrangement thereof.

Located within treatment room 400 are ion beam source 410, imaging system 420, signal generator 430, patient 440, table 450, receiver unit 460, cameras 470 and operator console 480. Signal generator 430, table 450 and receiver unit 460 are coupled to robotic arms 435, 455 and 465, respectively, to provide three-dimensional movement thereof. Markers 445, which may comprise implementations of marker 110 and/or marker 150, are implanted within patient 440.

Ion beam source 410 may comprise any source of proton, neutron, or heavier ion beams that is or becomes known. Generally, ion beam source 410 may comprise a linear accelerator coupled to a synchrotron to accelerate a beam of ions to a desired energy (e.g., 70 to 250 MeV). Ion beam source 410 may also include a scanning system to deflect the ion beam in two dimensions according to a treatment plan. Accordingly, the deflections may be controlled so as to scan the ion beam across a downstream two-dimensional area (i.e., a cross-section of the target).

Ions exit ion beam source 410 along beam axis 415, which may change direction if the beam is deflected as described above. The energies with which the ions exit ion beam source 410 determine the location of maximum energy deposition (i.e., the Bragg peak) associated with the ion beam. By varying the energies and employing the above-mentioned scanning method, the Bragg peak can be moved throughout a target in three dimensions.

Imaging system 420 includes a detector and an x-ray source mounted to a C-arm as is known in the art. The x-ray source may employ cathodes based on carbon nanotube or thermionic emission technology and/or a conventional x-ray tube. Imaging system 420 may rotates around an axis as illustrated by arrow 425, thereby rotating the detector and the x-ray source around the axis. This rotation, and the fixed relationship between the detector and the x-ray source, provide for creation of projection images of intervening tissue over a limited arc.

Digital tomosynthesis reconstruction algorithms, such as filtered back-projection algorithms, may be applied to these projection images to create cross-sectional images of the intervening tissue. For example, the projection images may be filtered with a Ram-Lak filter before back-projection. The cross-sectional images may be compared to the treatment plan to verify a position of patient 440 prior to ion therapy.

Embodiments may employ any other suitable imaging system that is or becomes known, including but not limited to a kilovoltage cone-beam system providing projection images spaced over an entire 360 degree arc, a computed tomography scanner, or a fixed ring of x-ray sources and associated detector as described in commonly-assigned co-pending application serial no. (Attorney docket no. 2009P05230US). As described therein, such x-ray sources may be disposed in a fixed relationship to each other and with respect to ion beam source 410.

Signal generator 430 generates a wireless signal to trigger electrical circuits within markers 445. As described above, the wireless signal may comprise a "switch-on" signal, a pulse having spectral components intended to cause resonance of electrical circuits within markers 445, or other suitable signal types. Support 435 may comprise a multi-jointed robotic arm to move signal generator 430 to a desired position with a desired orientation. In some embodiments, support 435 may be coupled to signal generator 430 via a ball joint or other type of coupling to provide roll, pitch and yaw movement of signal generator 430 with respect to support 435. Support 455 and table 450 may be similarly configured to move patient 440 with respect to ion beam source 410.

Receiver unit 460 supports two or more receivers such as receivers 240. Receiver unit 460, as well as its supported receivers, may be moved with respect to patient 440 (and markers 445) via robotic arm 465. Receiver unit 460 also includes sensors 467 which are detectable by cameras 470 to determine positions of the receivers with respect to cameras 470. Cameras 470 are fixed in room 400 and registered with the coordinate system of ion beam source 410. Accordingly, this registration may be used to determine locations of the receivers with respect to ion beam source 410.

Operator console 480 may be in communication with one or more of the above-described elements of FIG. 4 to provide control and/or monitoring thereof. Operator console 480 includes input device 481 for receiving instructions from an operator and output device 482, which may be a monitor for presenting beam characteristics of ion beam source 410, projection images acquired by imager 420, computed tomography images used for treatment planning, Bragg peak locations, cross-sectional digital tomosynthesis images, interfaces for receiving operator instructions, and/or operator alerts. According to some embodiments, output device 482 may present an alert notifying an operator of a positioning error prior to or during treatment delivery.

Input device 481 and output device 482 are coupled to processor 483 and storage 484. Processor 483 may execute program code to perform or cause to be performed any of the determinations and generations described herein. For example, processor 483 may determine locations of markers 445 based on signals received from receivers of receiver unit 460.

A hardware environment according to some embodiments may include fewer or more elements than those shown in FIG. 4. For example, unshown elements may be required to provide AC power, RF power, cooling, hydraulics, vacuum and/or other systems needed to operate the illustrated elements. In addition, embodiments are not limited to the illustrated elements and/or environment.

Returning to process 300, a patient is positioned according to a treatment plan prior to S301. The treatment plan may define multiple treatment fractions, each of which includes one or more discrete beams to be delivered. For each beam, the treatment plan may specify characteristics (e.g., energy, type, etc.) of the beam, a location of a Bragg peak associated with the beam, and expected locations of markers with respect to ion beam source 410. As described above, each beam can be static or moving, and/or may exhibit multiple radiation shapes delivered discretely (i.e., segments or ports) or continuously.

Positioning a patient according to the treatment plan may be performed by any suitable system. For example, imager 420 may acquire projection images of a patient volume of interest and processor 483 may create cross-sectional images therefrom. The cross-sectional images are compared with the treatment plan to determine whether the patient volume is positioned according to the treatment plan. The patient volume may include markers 445, and markers 445 may or may not appear in the cross-sectional images depending on the composition thereof. If markers 445 appear in the cross-sectional images, the locations of markers 445 may further assist in positioning the patient volume.

At S301, one or more wireless signals are generated to trigger one or more non-metal electrical circuits within respective biocompatible housings disposed in a patient volume. In one example of S301, each of markers 445 includes an electrical circuit associated with a unique resonant frequency. Signal generator 430 therefore generates a wireless signal including a significant spectral component corresponding to each resonant frequency. In response, each of markers 445 emits a respective wireless response signal.

Next, each receiver of receiver unit 460 receives each of the wireless response signals at S302 and locations of markers 445 are determined based on the signals at S303. According to some embodiments of S303, a time delay is measured between the end (or beginning) of the trigger signal and the end (or beginning) of each of the wireless response signals. Because the locations of signal generator 430 and the receivers are known, processor 483 may determine a location of a particular marker with respect to receiver unit 460 based on the time delay experienced by each receiver (or at least two receivers) for signals from the particular marker. The marker locations with respect to ion beam source 410 may then be determined based on their locations with respect to receiver unit 420, the location of receiver unit 420 in room 400 (determined, for example, by detecting sensors 467 with cameras 470) and the known transformation from the room coordinate system to the coordinate system of ion source 410.

S301 through S303 may proceed slightly differently in a case that markers 445 are implemented in accordance with marker 150 of FIG. 1B. For example, signal generator 430 may generate a trigger signal which causes active transmitters within markers 445 to emit unique and predetermined pulse trains. At S302, receivers of unit 420 receive the pulse trains and, at S303, processor 483 may determine locations of each marker 445 based on the difference in the reception time of a particular subsequence of its unique pulse train at two or more of the receivers. Again, these locations are determined with respect to receiver unit 420, and may be transformed to the coordinate system of ion beam source 410 as described above.

Next, at S304, it is determined whether the locations of the markers conform to the treatment plan. In this regard, the locations of markers 445 may serve as a proxy for the position (i.e., location and orientation) of a volume of interest within patient 140. The volume of interest may be a tumor or an organ to be avoided. Flow proceeds to S305 if the locations fail to conform to the treatment plan.

Either the Bragg peak, the patient volume, or both are moved at S305 based on the determined locations. The Bragg peak may be moved by changing characteristics of the ion beam (e.g., energy) and the patient volume may be moved by moving table 450 and/or repositioning patient 440 on table 450. The ion beam is then delivered to the patient volume at S306.

In some embodiments, flow returns to S301 after S305 to check whether the movement(s) performed in S305 resulted in the desired conformity of the locations with the treatment plan. If it is determined at S304 that the locations conform to the treatment plan, flow proceeds to S306 for delivery of the ion beam according to the currently-determined beam characteristics.

According to some embodiments, flow returns to S301 after S306 and proceeds as described above during delivery of the ion beam. Such an arrangement may provide monitoring of the locations of the markers during beam delivery. Delivery of the beam in these embodiments may terminate any time it is determined at S304 that the locations of the markers fail to conform to the treatment plan.

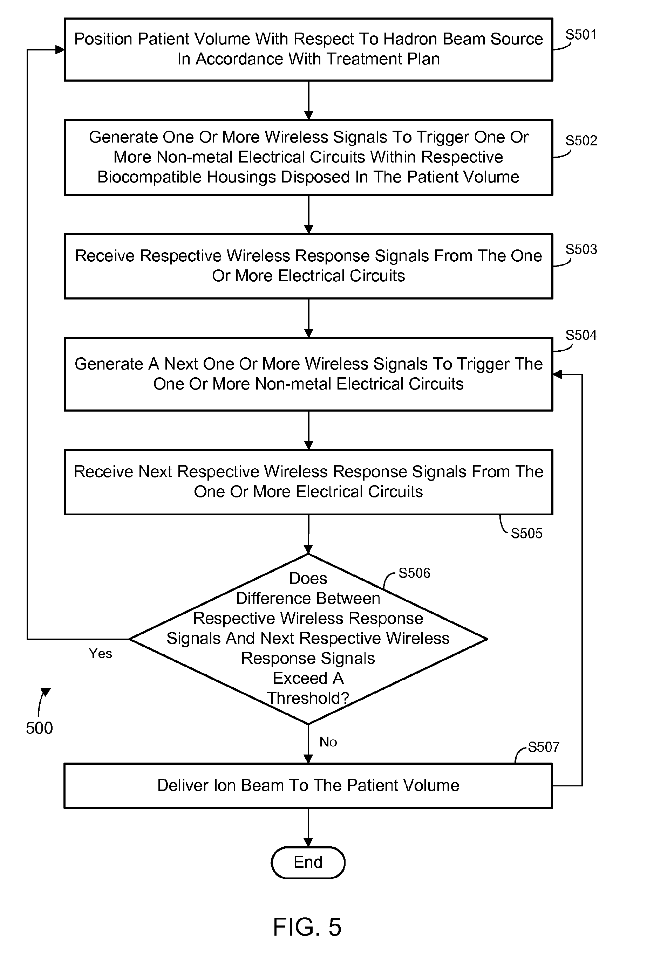

FIG. 5 is a flow diagram of process 500 according to some embodiments. Process 500 may provide benefits as mentioned herein without requiring specific determination of the locations of implanted markers. As such, implementations of process 500 may be technically simpler and less expensive than implementations of process 300.

Initially, at S501, a patient volume is positioned with respect to a ion beam source in accordance with a treatment plan. Such positioning may be performed as described above, typically using projection images and/or cross-sectional images of the patient volume acquired by an imaging system.

Next, at S502, one or more wireless signals are generated to trigger one or more non-metal electrical circuits within respective biocompatible housings disposed in the patient volume. Signal generator 430 may operate in any of the manners described above with respect to S301 to generate such signals.

Respective wireless response signals are received from the one or more electrical circuits at S503 as described above with respect to S302. In contrast to process 300, the received signals, which may be received individually or as a single composite signal, are not used to determine a location of the electrical circuits (i.e., markers). Rather, the received signals establish a baseline response that corresponds to proper positioning of the patient volume.

Flow then proceeds through S504, S505 and S506 to generate another wireless signal to trigger the electrical circuits, to receive next wireless response signals, and to determine whether a difference between the next wireless response signals and the response signals received at S503 exceeds a threshold. The determination at S506 is intended to determine whether the markers (and the patient volume of interest) have moved to an extent that requires repositioning of the patient volume. In some examples, the difference is determined to exceed the threshold if one of the markers has moved a significant distance and the other markers have remained substantially in place, or if several of the markers have moved slightly.

Accordingly, flow returns to S501 to reposition the patient volume if the determination of S506 is affirmative. In some embodiments, S501 may comprise changing a characteristic of a ion bean to be delivered based on the determined difference.

A ion beam is delivered to the patient volume at S507 if the determination at S506 is negative. Flow then returns to S504 and proceeds as described above during delivery of the ion beam to provide monitoring of the marker positions during beam delivery.

The embodiments described herein show a patient volume. However, embodiments are not restricted to patient volumes. Volumes of inanimate bodies such as phantoms or calibration equipment may be used in connection with embodiments, for example for research purposes or during commissioning or quality control of a system.

The several embodiments described herein are solely for the purpose of illustration. Therefore, persons in the art will recognize from this description that other embodiments may be practiced with various modifications and alterations.

## Claims

1. A system comprising:
a biocompatible housing; and
an electrical circuit disposed within the housing, the electrical circuit to emit a radiofrequency signal,
wherein the electrical circuit does not comprise a metal.

2. A system according to Claim 1, wherein the electrical circuit comprises:
a carbon nanocoil; and
a capacitor electrically coupled to the carbon nanocoil.

3. A system according to Claim 2., wherein the capacitor comprises carbon nanotubes.

4. A system according to Claim 1, further comprising:
a signal generator to generate a wireless signal to trigger the electrical circuit;
a receiver to receive a response signal generated by the triggered electrical circuit; and
a processor to determine a location of the biocompatible housing based on the response signal.

5. A system according to Claim 4, the processor to determine that the location conforms to a treatment plan, the system further comprising:
a ion beam source to deliver a ion beam associated with the treatment plan.

6. A system according to Claim 5, wherein the electrical circuit comprises:
a carbon nanocoil; and
a capacitor electrically coupled to the carbon nanocoil.

7. A system according to Claim 1, further comprising:
a signal generator to generate a wireless signal to trigger the electrical circuit;
a receiver to receive a wireless response signal generated by the triggered electrical circuit; and
a processor to determine a difference between the wireless response signal and a previous wireless response signal received from the electrical circuit.

8. A system according to Claim 7, the processor to determine that the difference exceeds a threshold, the system further comprising:
an imaging system to generate an image of a patient volume in which the biocompatible housing is located in response to the determination; and
a positioning system to change a position of the patient volume with respect to a ion beam source based on the image.

9. A system according to Claim 7, the processor to determine that the difference exceeds a threshold, the system further comprising:
an imaging system to generate an image of a patient volume in which the biocompatible housing is located in response to the determination; and
a positioning system to change one or more characteristics of a ion beam to be delivered by a ion beam source based on the image.

10. A system according to Claim 7, wherein the electrical circuit comprises:
a carbon nanocoil; and
a capacitor electrically coupled to the carbon nanocoil.

11. A method comprising:
generating a wireless signal to trigger an electrical circuit within a biocompatible housing disposed in a target volume; and
receiving a wireless response signal from the electrical circuit,
wherein the electrical circuit does not comprise a metal.

12. A method according to Claim 11, further comprising:
determining a location of the biocompatible housing based on the wireless response signal.

13. A method according to Claim 12, further comprising:
determining that the location conforms to a treatment plan; and
delivering a ion beam associated with the treatment plan to the target volume.

14. A method according to Claim 12, further comprising:
determining that the location does not conform to a treatment plan;
changing a relative position between the target volume and a Bragg peak of a ion beam to be delivered to the target volume based on the location; and
delivering the ion beam to the target volume according to the treatment plan.

15. A method according to Claim 11, further comprising:
generating a second wireless signal to trigger a second electrical circuit within a second biocompatible housing disposed in the target volume, the second electrical circuit not comprising a metal;
receiving a second wireless response signal from the second electrical circuit;
determining a location of the biocompatible housing based on the wireless response signal;
determining a second location of the second biocompatible housing based on the second wireless response signal;
determining that the location and the second location conform to a treatment plan; and
delivering a ion beam associated with the treatment plan to the target volume.

16. A method according to Claim 11, further comprising:
generating a second wireless signal to trigger a second electrical circuit within a second biocompatible housing disposed in the target volume, the second electrical circuit not comprising a metal;
receiving a second wireless response signal from the second electrical circuit;
determining a location of the biocompatible housing based on the wireless response signal;
determining a second location of the second biocompatible housing based on the second wireless response signal;
determining that the location and the second location do not conform to a treatment plan;
changing a relative position between the target volume and a Bragg peak of a ion beam to be delivered to the target volume based on the location; and
delivering the ion beam to the target volume.

17. A method according to Claim 11, further comprising:
determining a difference between the wireless response signal and a previous wireless response signal received from the electrical circuit; and
determining whether the difference exceeds a threshold.

18. A method according to Claim 17, further comprising:
delivering a ion beam to the target volume if the difference does not exceed the threshold.

19. A method according to Claim 17, further comprising:
if the difference exceeds the threshold, generating an image of the target volume;
changing a relative position between the target volume and a Bragg peak of a ion beam to be delivered to the target volume based on the image; and
delivering the ion beam to the target volume.

20. A method according to Claim 17, the wireless signal to trigger a second electrical circuit within a second biocompatible housing disposed in the target volume,
wherein receiving the wireless response signal comprises receiving a wireless combined signal comprising the wireless response signal and a second wireless response signal generated by the second electrical circuit, and
wherein determining the difference comprises determining the difference between the wireless combined signal and a previously-received wireless combined signal.

21. A method according to Claim 20, further comprising:
if the difference exceeds the threshold, generating an image of the target volume; and
changing a relative position between the target volume and a Bragg peak of a ion beam to be delivered to the target volume based on the image; and
delivering the ion beam to the target volume.

22. A particle therapy system comprising:
an output unit for inducing generation of a wireless signal to trigger an electrical circuit within a biocompatible housing disposed in a target volume;
an input unit for receiving a wireless response signal from the electrical circuit, wherein the electrical circuit does not comprise a metal;
an evaluation unit for determining a location of the biocompatible housing based on the wireless response signal;
an ion beam delivering unit for delivering a ion beam depending on the determined location of the biocompatible housing.

23. A particle therapy system according to claim 22, wherein the ion beam delivering unit is adapted to change a relative position between the target volume and a Bragg peak of the ion beam to be delivered to the target volume based on the location determined location of the biocompatible housing.

24. A particle therapy system according to Claim 22, wherein the evaluation unit is adapted to determine a difference between the wireless response signal and a previous wireless response signal received from the electrical circuit.

25. A particle therapy system according to Claim 24, particle therapy system further comprising:
an imaging unit for generating an image of the target volume, wherein the particle therapy system is adapted to generate an image of the target volume if the difference exceeds a threshold.

26. A particle therapy system according to Claim 22, particle therapy system further comprising:
an imaging unit for generating an image of the target volume, wherein imaging is performed depending on the determined location of the biocompatible housing.
